# EUROPEAN PATENT SPECIFICATION

(11) **EP 3 468 548 B1**
(45) Date of publication and mention of the grant of the patent: **04.08.2021**
(21) Application number: 17813938.2
(22) Date of filing: 13.06.2017
(51) Int. Cl.: A61K 31/435, A61K 31/437, C07D 417/02, C07D 417/08, C07D 471/04, A61P 35/00

(54) **METHODS OF TREATING PANCREATIC CANCER**
VERFAHREN ZUR BEHANDLUNG VON BAUCHSPEICHELDRÜSENKREBS
MÉTHODES DE TRAITEMENT DU CANCER DU PANCRÉAS

(30) Priority: 13.06.2016 US 201662349217 P; 01.09.2016 US 201662382689 P
(43) Date of publication of application: 17.04.2019
(73) Proprietor: ChemoCentryx, Inc., Mountain View California 94043 (US)
(72) Inventor: BEKKER, Petrus, Mountain View CA 94043 (US); MIAO, Shichang, Foster City CA 94404 (US); CHARO, Israel, Mountain View CA 94043 (US); SCHALL, Tom, Mountain View CA 94043 (US)
(74) Representative: Parchmann, Stefanie
(86) International application number: PCT/US2017/037264
(87) International publication number: WO 2017/218544

(56) References cited:
- WO-A1-2009/009740
- US-A1- 2010 249 118
- US-A1- 2012 122 860
- US-A1- 2014 031 348
- A MERCALLI ET AL: "A preclinical evaluation of pemetrexed and irinotecan combination as second-line chemotherapy in pancreatic cancer", BRITISH JOURNAL OF CANCER, vol. 96, no. 9, 10 April 2007 (2007-04-10) , pages 1358-1367, XP55651816, GB ISSN: 0007-0920, DOI: 10.1038/sj.bjc.6603726

## Description

### FIELD

The present disclosure pertains to a compound for use in treating pancreatic cancer and limiting over-expression of oncogenes, activating tumor suppressor genes or regulating signaling proteins in patients and to pharmaceutical combinations as described herein.

### BACKGROUND

Cancer is a significant health problem throughout the world. Although advances have been made in detection and therapy of cancer, no vaccine or other universally successful method for prevention and/ or treatment is currently available.

Current therapies, which are generally based on a combination of chemotherapy or surgery and radiation, continue to prove inadequate in many patients.

Located in the upper abdomen in the retroperitoneum, the pancreas is associated intimately with many major structures including the portal vein, stomach, duodenum, common bile duct and the superior mesenteric artery.

Pancreatic cancer is the fifth leading cause of cancer death in the United States. It is more common among men, and men between the ages of 60 and 70 are most at risk. As the tumor grows, the patient's symptoms result from tumor infiltration of surrounding structure causing pain, nausea, vomiting, weight loss and jaundice. The latter condition presents symptoms in no more than one half of the patients.

Once tumor infiltration occurs other structures such as the portal vein become affected and this precludes curative resectioning of the pancreas.

Effective treatment of pancreas cancer is delayed frequently for several months. This delay has profound implications, since metastatic spread to the liver or lymph nodes has been observed at a time of diagnosis in 60% of patients, and this factor diminishes the prospect for long-term survival. Also, the carcinoma of the pancreas is asymptomatic in its early stage. The most common symptoms at later stage are weight loss, abdominal pain, and jaundice. Weight loss, the causes of which are not fully understood, usually is significant. Jaundice occurs if the cancer blocks the common bile duct. By the time the malignant tumor is identified, it often has spread (metastasized) to other parts of the body. The median survival is little more than six months from the time of diagnosis.

Current therapies for this common and difficult-to-treat disease include surgery and/or chemotherapy. Often the tumor cannot be removed by surgery, either because it has invaded vital structures that cannot be removed or because it has spread to distant sites.

WO 2009/009740 A1 describes aryl sulfonamide compounds that modulate CCR2 chemokine ligand activity and their use in treating CCR2-mediated diseases, for example cancer. Mercalli et al., British Journal of Cancer 96(9):1358-1367 describes the preclinical evalution of a pemetrexed and irinotecan combination as second-line chemotherapy in pancreatic cancer.

Accordingly, there is a need in the art for improved treating primary and metastatic pancreatic cancers.

### Figures

Figure 1 represents the mean plasma concentration of Compound Ib following i.v. in dog dosing of Compound lb.
Figure 2 represents the mean plasma concentration of Compound Ib following p.o. dosing in dog of Compound lb.

### Summary

The present disclosure provides compounds that modulate CCR2 chemokine ligand activity and can be used in methods of treating pancreatic cancer. Accordingly, the compounds of the present disclosure are compounds that modulate at least one function or characteristic of mammalian CCR2, for example, a human CCR2 protein.

Thus, the present disclosure describes compounds for use in treating pancreatic cancer in patients including administering the compounds and pharmaceutical combinations as described herein. Also described are methods of limiting over-expression of oncogenes, activating tumor suppressor genes, and regulating signaling proteins that include administering to a patient in need thereof an effective amount of any of the compounds or pharmaceutical combinations as described herein.

One embodiment of the present invention is a compound of Formula I: 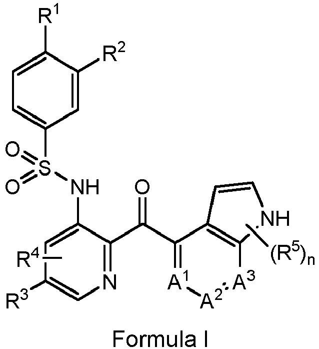 or a pharmaceutically acceptable salt thereof for use in treating pancreatic cancer or in controlling an adenocarcinoma, wherein R¹ is halogen or C₁₋₆ alkyl; R² is hydrogen, halogen, C₁₋₆ alkyl, C₁₋₆ alkoxy, C₁₋₆ haloalkyl, C₁₋₆ haloalkoxy, or -CN; R³ is hydrogen, halogen, or C₁₋₆ alkyl; R⁴ is hydrogen, halogen, or C₁₋₆ alkyl; each R⁵ is independently C₁₋₆ alkyl, -OH, or-NH₂; n is 0, 1, 2, or 3; and each of A¹, A², and A³ is -CH- or -N-, where at least one of A¹, A², or A³ is -N-. In one embodiment, R¹ is halogen or methyl; R² is halogen or C₁₋₆ haloalkyl; R³ is halogen or C₁₋₆ alkyl; R⁴ is hydrogen; n is 0; A¹ is -CH- or-N-; A² is -CH-; and A³ is - N-. In one embodiment, R¹ is halogen or methyl; R² is C₁₋₃ haloalkyl; R³ is halogen or C₁₋₃ alkyl; R⁴ is hydrogen; n is 0; A¹ is -CH- or -N-; A² is -CH-; and A³ is -N-.

In one embodiment, the compound is selected from the group consisting of: 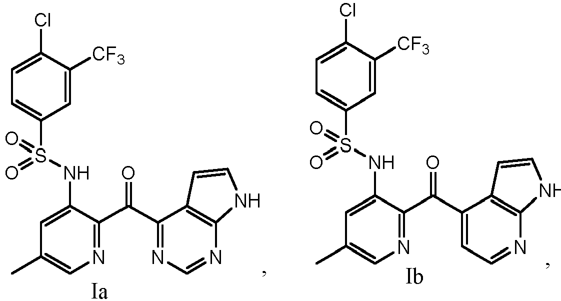 and 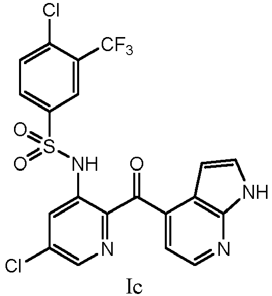 or a pharmaceutically acceptable salt thereof.

In one embodiment, the compound is the compound of Formula Ib: 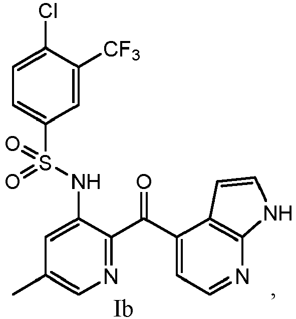 or a pharmaceutically acceptable salt thereof.

In one embodiment, the pancreatic cancer is Stage I, II, III, or IV.

In one embodiment, the treatment provides one or more of a decrease in tumor size, a suppression or decrease in tumor growth, no new tumor formation, a decrease in new tumor formation, an increase in survival or progression-free survival, no metastases, an increase in treatment options, delay in time from surgery to recurrence, reduction in jaundice, suppression of spread to liver, reduction in pain, improved appetite, improved digestion, reduction of gallbladder size, and reduced incidence of blood clots.

In one embodiment, the patient achieves a complete response. In one embodiment, the patient achieves a partial response. In one embodiment, the patient achieves stable disease. In one embodiment, the patient achieves a slower progressive disease.

In one embodiment, the patient suffers from one or more of pancreatic adenocarcinoma, non-resectable pancreatic cancer, locally advanced pancreatic cancer, borderline resectable pancreatic cancer, locally advanced pancreatic ductal adenocarcinoma, borderline resectable pancreatic ductal adenocarcinoma, metastatic pancreatic cancer, chemotherapy-resistant pancreatic cancer, pancreatic ductal adenocarcinoma, squamous pancreatic cancer, pancreatic progenitor, immunogenic pancreatic cancer, aberrantly differentiated endocrine exocrine (ADEX) tumors, an exocrine pancreatic cancer, pancreatic intraepithelial neoplasia, intraductal papillary mucinous neoplasms, mucinous cystic neoplasms, mucinous pancreas cancer, adenosquamous carcinoma, signet ring cell carcinoma, hepatoid carcinoma, colloid carcinoma, undifferentiated carcinoma, undifferentiated carcinomas with osteoclast-like giant cells, a pancreatic cystic neoplasm, an islet cell tumor, a pancreas endocrine tumor, or a pancreatic neuroendrocrine tumor.

In one embodiment, the compound of Formula I is provided as a pharmaceutical composition for oral administration.

In one embodiment, the compound is administered once a day. In one embodiment, the compound is administered twice a day.

In one embodiment, the treatment includes administering to the patient one or more additional therapeutic compounds. In one embodiment, the one or more additional therapeutic compound is selected from one or more of a Btk tyrosine kinase inhibitor, an Erbb2 tyrosine kinase receptor inhibitor; an Erbb4 tyrosine kinase receptor inhibitor, an mTOR inhibitor, a thymidylate synthase inhibitor, an EGFR tyrosine kinase receptor inhibitor, an epidermal growth factor antagonist, a Fyn tyrosine kinase inhibitor, a kit tyrosine kinase inhibitor, a Lyn tyrosine kinase inhibitor, a NK cell receptor modulator, a PDGF receptor antagonist, a PARP inhibitor, a poly ADP ribose polymerase inhibitor, a poly ADP ribose polymerase 1 inhibitor, a poly ADP ribose polymerase 2 inhibitor, a poly ADP ribose polymerase 3 inhibitor, a galactosyltransferase modulator, a dihydropyrimidine dehydrogenase inhibitor, an orotate phosphoribosyltransferase inhibitor, a telomerase modulator, a mucin 1 inhibitor, a mucin inhibitor, a secretin agonist, a TNF related apoptosis inducing ligand modulator, an IL17 gene stimulator, an interleukin 17E ligand, a neurokinin receptor agonist, a cyclin G1 inhibitor, a checkpoint inhibitor, a PD-1 inhibitor, a PD-L1 inhibitor, a CTLA4 inhibitor, a topoisomerase I inhibitor, an Alk-5 protein kinase inhibitor, a connective tissue growth factor ligand inhibitor, a notch-2 receptor antagonist, a notch-3 receptor antagonist, a hyaluronidase stimulator, a MEK-1 protein kinase inhibitor; MEK-2 protein kinase inhibitor, a GM-CSF receptor modulator; TNF alpha ligand modulator, a mesothelin modulator, an asparaginase stimulator, a caspase-3 stimulator; caspase-9 stimulator, a PKN3 gene inhibitor, a hedgehog protein inhibitor; smoothened receptor antagonist, an AKT1 gene inhibitor, a DHFR inhibitor, a thymidine kinase stimulator, a CD29 modulator, a fibronectin modulator, an interleukin-2 ligand, a serine protease inhibitor, a D40LG gene stimulator; TNFSF9 gene stimulator, a 2-oxoglutarate dehydrogenase inhibitor, a TGF-beta type II receptor antagonist, an Erbb3 tyrosine kinase receptor inhibitor, a cholecystokinin CCK2 receptor antagonist, a Wilms tumor protein modulator, a Ras GTPase modulator, an histone deacetylase inhibitor, a cyclin-dependent kinase 4 inhibitor A modulator, an estrogen receptor beta modulator, a 4-1BB inhibitor, a 4-1BBL inhibitor, a PD-L2 inhibitor, a B7-H3 inhibitor, a B7-H4 inhibitor, a BTLA inhibitor, a HVEM inhibitor, aTIM3 inhibitor, a GAL9 inhibitor, a LAG3 inhibitor, a VISTA inhibitor, a KIR inhibitor, a 2B4 inhibitor, a CD160 inhibitor and a CD66e modulator. In one embodiment, the one or more additional therapeutic compound is selected from one or more of bavituximab, IMM-101, CAP1-6D, Rexin-G , genistein, CVac, MM-D37K, PCI-27483, TG-01, mocetinostat, LOAd-703, CPI-613, upamostat, CRS-207, NovaCaps, trametinib, Atu-027, sonidegib, GRASPA, trabedersen, nastorazepide, Vaccell, oregovomab, istiratumab, refametinib, regorafenib, lapatinib, selumetinib, rucaparib, pelareorep, tarextumab, PEGylated hyaluronidase, varlitinib, aglatimagene besadenovec, GBS-01, GI-4000, WF-10, galunisertib, afatinib, RX-0201, FG-3019, pertuzumab, DCVax-Direct, selinexor, glufosfamide, virulizin, yttrium (90Y) clivatuzumab tetraxetan, brivudine, nimotuzumab, algenpantucel-L, tegafur + gimeracil + oteracil potassium + calcium folinate, olaparib, ibrutinib, pirarubicin, Rh-Apo2L, tertomotide, tegafur + gimeracil + oteracil potassium, tegafur + gimeracil + oteracil potassium, masitinib, Rexin-G, mitomycin, erlotinib, adriamycin, dexamethasone, vincristine, cyclophosphamide, fluorouracil, topotecan, taxol, interferons, platinum derivatives, taxane, paclitaxel, vinca alkaloids, vinblastine, anthracyclines, doxorubicin, epipodophyllotoxins, etoposide, cisplatin, rapamycin, methotrexate, actinomycin D, dolastatin 10, colchicine, emetine, trimetrexate, metoprine, cyclosporine, daunorubicin, teniposide, amphotericin, alkylating agents, chlorambucil, 5-fluorouracil, campthothecin, metronidazole, Gleevec, Avastin, Vectibix, abarelix, aldesleukin, alemtuzumab, alitretinoin, allopurinol, altretamine, amifostine, anastrozole, arsenic trioxide, asparaginase, azacitidine, AZD9291, BCG Live, bevacuzimab, fluorouracil, bexarotene, bleomycin, bortezomib, busulfan, calusterone, capecitabine, camptothecin, carboplatin, carmustine, celecoxib, cetuximab, chlorambucil, cladribine, clofarabine, cyclophosphamide, cytarabine, dactinomycin, darbepoetin alfa, daunorubicin, denileukin, dexrazoxane, docetaxel, doxorubicin (neutral), doxorubicin hydrochloride, dromostanolone propionate, epirubicin, epoetin alfa, estramustine, etoposide phosphate, etoposide, exemestane, filgrastim, floxuridine fludarabine, fulvestrant, gefitinib, gemcitabine, gemtuzumab, goserelin acetate, histrelin acetate, hydroxyurea, ibritumomab, idarubicin, ifosfamide, imatinib mesylate, interferon alfa-2a, interferon alfa-2b, irinotecan, lenalidomide, letrozole, leucovorin, leuprolide acetate, levamisole, lomustine, megestrol acetate, melphalan, mercaptopurine, 6-MP, mesna, methotrexate, methoxsalen, mitomycin C, mitotane, mitoxantrone, nandrolone, nelarabine, nofetumomab, oprelvekin, oxaliplatin, paclitaxel, palifermin, pamidronate, pegademase, pegaspargase, pegfilgrastim, pemetrexed disodium, pentostatin, pipobroman, plicamycin, porfimer sodium, procarbazine, quinacrine, rasburicase, rituximab, rociletinib, sargramostim, sorafenib, streptozocin, sunitinib maleate, talc, tamoxifen, temozolomide, teniposide, VM-26, testolactone, thioguanine, 6-TG, thiotepa, topotecan, toremifene, tositumomab, trastuzumab, tretinoin, ATRA, uracil mustard, valrubicin, vinblastine, vincristine, vinorelbine, zoledronate, zoledronic acid, pembrolizumab, nivolumab, IBI-308, mDX-400, BGB-108, MEDI-0680, SHR-1210, PF-06801591, PDR-001, GB-226, STI-1110, durvalumab, atezolizumab, avelumab, BMS-936559, ALN-PDL, TSR-042, KD-033, CA-170, STI-1014, FOLFIRINOX and KY-1003. In one embodiment, the one or more additional therapeutic compound is FOLFIRINOX. In one embodiment, the one or more additional therapeutic compounds are gemcitabine and paclitaxel. In one embodiment, the one or more additional therapeutic compounds are gemcitabine and nab-paclitaxel.

In one embodiment of the present invention, the compound of Formula I is a compound of Formula Ib: 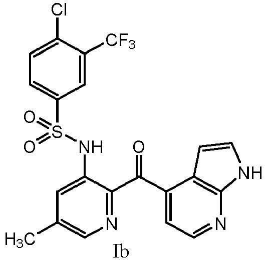 or a pharmaceutically acceptable salt thereof.

In one embodiment, the effective amount is from 50 mg to 300 mg. In one embodiment, the effective amount is 150 mg. One embodiment of the present invention is a pharmaceutical combination comprising a compound of Formula I: 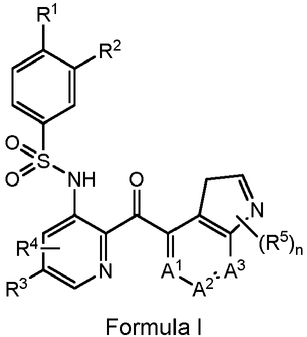 or a pharmaceutically acceptable salt thereof, wherein R¹ is halogen or C₁₋₆ alkyl; R² is hydrogen, halogen, C₁₋₆ alkyl, C₁₋₆ alkoxy, C₁₋₆ haloalkyl, C₁₋₆ haloalkoxy, or -CN; R³ is hydrogen, halogen, or C₁₋₆ alkyl; R⁴ is hydrogen, halogen, or C₁₋₆ alkyl; each R⁵ is independently C₁₋₆ alkyl, -OH, or -NH₂; n is 0, 1, 2, or 3; each of A¹, A², and A³ is -CH- or - N- where at least one of A¹, A², or A³ is -N-; and one or more additional therapeutic compounds. The one or more additional therapeutic compound is selected from one or more of a Btk tyrosine kinase inhibitor, an Erbb2 tyrosine kinase receptor inhibitor; an Erbb4 tyrosine kinase receptor inhibitor, an mTOR inhibitor, a thymidylate synthase inhibitor, an EGFR tyrosine kinase receptor inhibitor, an epidermal growth factor antagonist, a Fyn tyrosine kinase inhibitor, a kit tyrosine kinase inhibitor, a Lyn tyrosine kinase inhibitor, a NK cell receptor modulator, a PDGF receptor antagonist, a PARP inhibitor, a poly ADP ribose polymerase inhibitor, a poly ADP ribose polymerase 1 inhibitor, a poly ADP ribose polymerase 2 inhibitor, a poly ADP ribose polymerase 3 inhibitor, a galactosyltransferase modulator, a dihydropyrimidine dehydrogenase inhibitor, an orotate phosphoribosyltransferase inhibitor, a telomerase modulator, a mucin 1 inhibitor, a mucin inhibitor, a secretin agonist, a TNF related apoptosis inducing ligand modulator, an IL17 gene stimulator, an interleukin 17E ligand, a neurokinin receptor agonist, a cyclin G1 inhibitor, a checkpoint inhibitor, a PD-1 inhibitor, a PD-L1 inhibitor, a CTLA4 inhibitor, a topoisomerase I inhibitor, an Alk-5 protein kinase inhibitor, a connective tissue growth factor ligand inhibitor, a notch-2 receptor antagonist, a notch-3 receptor antagonist, a hyaluronidase stimulator, a MEK-1 protein kinase inhibitor; MEK-2 protein kinase inhibitor, a GM-CSF receptor modulator; TNF alpha ligand modulator, a mesothelin modulator, an asparaginase stimulator, a caspase-3 stimulator; caspase-9 stimulator, a PKN3 gene inhibitor, a hedgehog protein inhibitor; smoothened receptor antagonist, an AKT1 gene inhibitor, a DHFR inhibitor, a thymidine kinase stimulator, a CD29 modulator, a fibronectin modulator, an interleukin-2 ligand, a serine protease inhibitor, a D40LG gene stimulator; TNFSF9 gene stimulator, a 2-oxoglutarate dehydrogenase inhibitor, a TGF-beta type II receptor antagonist, an Erbb3 tyrosine kinase receptor inhibitor, a cholecystokinin CCK2 receptor antagonist, a Wilms tumor protein modulator, a Ras GTPase modulator, an histone deacetylase inhibitor, a cyclin-dependent kinase 4 inhibitor A modulator, an estrogen receptor beta modulator, a 4-1BB inhibitor, a 4-1BBL inhibitor, a PD-L2 inhibitor, a B7-H3 inhibitor, a B7-H4 inhibitor, a BTLA inhibitor, a HVEM inhibitor, aTIM3 inhibitor, a GAL9 inhibitor, a LAG3 inhibitor, a VISTA inhibitor, a KIR inhibitor, a 2B4 inhibitor, a CD160 inhibitor and a CD66e modulator. In one embodiment, the one or more additional therapeutic compound is selected from one or more of bavituximab, IMM-101, CAP1-6D, Rexin-G , genistein, CVac, MM-D37K, PCI-27483, TG-01, mocetinostat, LOAd-703, CPI-613, upamostat, CRS-207, novaCaps, trametinib, Atu-027, sonidegib, GRASPA, trabedersen, nastorazepide, Vaccell , oregovomab, istiratumab, refametinib, regorafenib, lapatinib, selumetinib, rucaparib, pelareorep, tarextumab, PEGylated hyaluronidase, varlitinib, aglatimagene besadenovec, GBS-01, GI-4000, WF-10, galunisertib, afatinib, RX-0201, FG-3019, pertuzumab, DCVax-Direct, selinexor, glufosfamide, virulizin, yttrium (90Y) clivatuzumab tetraxetan, brivudine, nimotuzumab, algenpantucel-L, tegafur + gimeracil + oteracil potassium + calcium folinate, olaparib, ibrutinib, pirarubicin, Rh-Apo2L, tertomotide, tegafur + gimeracil + oteracil potassium, tegafur + gimeracil + oteracil potassium, masitinib, Rexin-G, mitomycin, erlotinib , adriamycin, dexamethasone, vincristine, cyclophosphamide, fluorouracil, topotecan, taxol, interferons, platinum derivatives, taxane, paclitaxel, vinca alkaloids, vinblastine, anthracyclines, doxorubicin, epipodophyllotoxins, etoposide, cisplatin, rapamycin, methotrexate, actinomycin D, dolastatin 10, colchicine, emetine, trimetrexate, metoprine, cyclosporine, daunorubicin, teniposide, amphotericin, alkylating agents, chlorambucil, 5-fluorouracil, campthothecin, cisplatin, metronidazole, Gleevec, Avastin, Vectibix, abarelix, aldesleukin, alemtuzumab, alitretinoin, allopurinol, altretamine, amifostine, anastrozole, arsenic trioxide, asparaginase, azacitidine, AZD9291, BCG Live, bevacuzimab, fluorouracil, bexarotene, bleomycin, bortezomib, busulfan, calusterone, capecitabine, camptothecin, carboplatin, carmustine, celecoxib, cetuximab, chlorambucil, cladribine, clofarabine, cyclophosphamide, cytarabine, dactinomycin, darbepoetin alfa, daunorubicin, denileukin, dexrazoxane, docetaxel, doxorubicin (neutral), doxorubicin hydrochloride, dromostanolone propionate, epirubicin, epoetin alfa, estramustine, etoposide phosphate, etoposide, exemestane, filgrastim, floxuridine fludarabine, fulvestrant, gefitinib, gemcitabine, gemtuzumab, goserelin acetate, histrelin acetate, hydroxyurea, ibritumomab, idarubicin, ifosfamide, imatinib mesylate, interferon alfa-2a, interferon alfa-2b, irinotecan, lenalidomide, letrozole, leucovorin, leuprolide acetate, levamisole, lomustine, megestrol acetate, melphalan, mercaptopurine, 6-MP, mesna, methotrexate, methoxsalen, mitomycin C, mitotane, mitoxantrone, nandrolone, nelarabine, nofetumomab, oprelvekin, oxaliplatin, paclitaxel, palifermin, pamidronate, pegademase, pegaspargase, pegfilgrastim, pemetrexed disodium, pentostatin, pipobroman, plicamycin, porfimer sodium, procarbazine, quinacrine, rasburicase, rituximab, rociletinib, sargramostim, sorafenib, streptozocin, sunitinib maleate, talc, tamoxifen, temozolomide, teniposide, VM-26, testolactone, thioguanine, 6-TG, thiotepa, topotecan, toremifene, tositumomab, trastuzumab, tretinoin, ATRA, uracil mustard, valrubicin, vinblastine, vincristine, vinorelbine, zoledronate, zoledronic acid, pembrolizumab, nivolumab, IBI-308, mDX-400, BGB-108, MEDI-0680, SHR-1210, PF-06801591, PDR-001, GB-226, STI-1110, durvalumab, atezolizumab, avelumab, BMS-936559, ALN-PDL, TSR-042, KD-033, CA-170, STI-1014, FOLFIRINOX and KY-1003. In one embodiment, the one or more additional therapeutic compound is FOLFIRINOX. In one embodiment, the one or more additional therapeutic compound are gemcitabine and paclitaxel. In one embodiment, the one or more additional therapeutic compound is gemcitabine and nab-paclitaxel. In one embodiment, the combination comprises a fixed dose combination or separate doses. In one embodiment, R¹ is halogen or methyl; R² is halogen or C₁₋₆ haloalkyl; R³ is halogen or C₁₋₆ alkyl; R⁴ is hydrogen; n is 0; A¹ is -CH- or -N-; A² is -CH-; and A³ is -N-. In one embodiment, R¹ is halogen or methyl; R² is C₁₋₃ haloalkyl; R³ is halogen or C₁₋₃ alkyl; R⁴ is hydrogen; n is 0; A¹ is -CH- or -N-; A² is -CH-; and A³ is -N-. In one embodiment, the compound of Formula I in the combination is selected from: 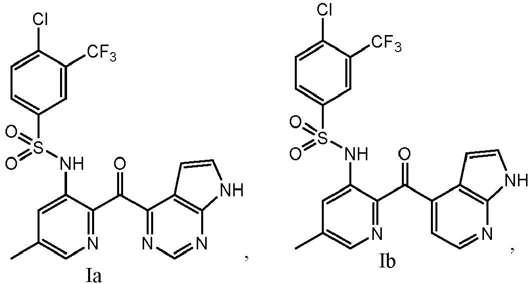 and 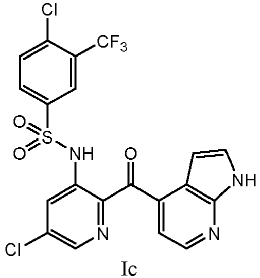 or a pharmaceutically acceptable salt thereof. In one embodiment, the compound of Formula I in the combination is the compound of Formula Ib: 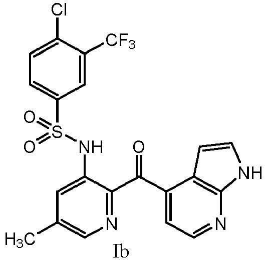 or a pharmaceutically acceptable salt thereof.

In one embodiment, the effective amount is from 50 mg to 300 mg. In one embodiment, the effective amount is 150 mg.

### DETAILED DESCRIPTION

The present disclosure is directed to compounds for use in treating pancreatic cancer in patients by administering the compounds and pharmaceutical combinations as described herein. Also described are methods of limiting over-expression of oncogenes, activating tumor suppressor genes, or regulating signaling proteins comprising administering to a patient in need thereof an effective amount of any of the compounds or pharmaceutical combinations as described herein. Accordingly, the present disclosure describes compounds which modulate at least one function or characteristic of mammalian CCR2, for example, a human CCR2 protein.

### Abbreviations and definitions

The following definitions are meant to clarify the terms defined.

"Alkyl" by itself or as part of another substituent refers to a hydrocarbon group which may be linear, cyclic, or branched or a combination thereof having the number of carbon atoms designated (i.e., C₁₋₈ means one to eight carbon atoms). Examples of alkyl groups include methyl, ethyl, n-propyl, isopropyl, n-butyl, t-butyl, isobutyl, sec-butyl, cyclohexyl, cyclopentyl, (cyclohexyl)methyl, cyclopropylmethyl, bicyclo[2.2.1]heptane, bicyclo[2.2.2]octane, etc.

"Alkoxy" refers to -O-alkyl. Examples of an alkoxy group include methoxy, ethoxy, n-propoxy etc.

"Clinical benefit" refers to a phrase used by doctors and/or clinicians treating cancer. The term encompasses any appreciated or perceived benefit encountered by a patient during therapy. As used herein, one or more of clinical benefit is one or more of decrease in tumor size, suppression or decrease of tumor growth, delayed time to progression, no new tumor or lesion, a decrease in new tumor formation, an increase in survival or progression-free survival, no metastases, increase in treatment options, delay in time from surgery to recurrence, reduction in jaundice, suppression of spread to liver, reduction in pain, improved appetite, improved digestion, reduction of gallbladder size, and reduced incidence of blood clots.

"Complete response" refers to a response which results in complete eradication of noninvasive or invasive cancers.

"FOLFIRINOX" refers to a chemotherapy regimen for treatment of advanced pancreatic cancer. It is made up of the following four drugs:
a) FOL - folinic acid (leucovorin), a vitamin B derivative that modulates/potentiates/reduces the side effects of fluorouracil;
b) F - fluorouracil (5-FU), a pyrimidine analog and antimetabolite which incorporates into the DNA molecule and stops DNA synthesis;
c) IRIN - irinotecan (Camptosar), a topoisomerase inhibitor, which prevents DNA from uncoiling and duplicating; and
d) OX - oxaliplatin (Eloxatin), a platinum-based antineoplastic agent, which inhibits DNA repair and/or DNA synthesis.

"Halo" or "halogen", by itself or as part of a substituent refers to a chlorine, bromine, iodine, or fluorine atom.

"Haloalkyl", as a substituted alkyl group, refers to a monohaloalkyl or polyhaloalkyl group, most typically substituted with from 1-3 halogen atoms. Examples include 1-chloroethyl, 3-bromopropyl, and trifluoromethyl.

"Heteroatom" is meant to include oxygen (O), nitrogen (N), sulfur (S) and silicon (Si).

"Partial response" refers to a response which results in at least 30% reduction in tumor size as compared to baseline, namely prior to administration of compound.

"Pharmaceutically acceptable" carrier, diluent, or excipient is a carrier, diluent, or excipient compatible with the other ingredients of the formulation and not deleterious to the recipient thereof.

"Pharmaceutically acceptable salt" refers to a salt which is acceptable for administration to a patient, such as a mammal (e.g., salts having acceptable mammalian safety for a given dosage regime). Such salts can be derived from pharmaceutically acceptable inorganic or organic bases and from pharmaceutically acceptable inorganic or organic acids, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Salts derived from pharmaceutically acceptable inorganic bases include aluminum, ammonium, calcium, copper, ferric, ferrous, lithium, magnesium, manganic, manganous, potassium, sodium, and zinc salts. Salts derived from pharmaceutically-acceptable organic bases include salts of primary, secondary, tertiary and quaternary amines, including substituted amines, cyclic amines, and naturally-occurring amines, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, and tromethamine. When compounds of the present disclosure contain relatively basic functionalities, acid addition salts can be obtained by contacting the neutral form of such compounds with a sufficient amount of the desired acid, either neat or in a suitable inert solvent. Salts derived from pharmaceutically acceptable acids include acetic, ascorbic, benzenesulfonic, benzoic, camphosulfonic, citric, ethanesulfonic, fumaric, gluconic, glucoronic, glutamic, hippuric, hydrobromic, hydrochloric, isethionic, lactic, lactobionic, maleic, malic, mandelic, methanesulfonic, mucic, naphthalenesulfonic, nicotinic, nitric, pamoic, pantothenic, phosphoric, succinic, sulfuric, tartaric, and p-toluenesulfonic salts.

Also included are salts of amino acids such as arginate, and salts of organic acids like glucuronic or galactunoric acids (see, for example, Berge, S. M. et al, "Pharmaceutical Salts", J. Pharmaceutical Science, 1977, 66:1-19). Certain specific compounds of the present disclosure contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present disclosure.

"Progressive disease" refers to at least 20% growth in the size of a tumor or spread of a tumor since the beginning of treatment.

"Salt thereof" refers to either a compound formed when the hydrogen of an acid is replaced by a cation, such as a metal cation or an organic cation, or a compound formed from a base protonated with a counter-ion. Preferably, the salt is a pharmaceutically-acceptable salt, although this is not required for salts of intermediate compounds which are not intended for administration to a patient.

"Stable disease" refers to cancer that is neither increasing nor decreasing in extent or severity.

"Therapeutically effective amount" refers to an amount sufficient to effect treatment when administered to a patient in need of treatment.

"Treating" or "treatment" as used herein refers to the treating or treatment of a disease or medical condition in a patient, such as a mammal (particularly a human or a companion animal) which includes ameliorating the disease or medical condition, i.e., eliminating or causing regression of the disease or medical condition in a patient; suppressing the disease or medical condition, i.e., slowing or arresting the development of the disease or medical condition in a patient; or alleviating the symptoms of the disease or medical condition in a patient.

Certain compounds of the present disclosure can exist in unsolvated forms as well as solvated forms, including hydrated forms. Certain compounds of the present disclosure may exist in multiple crystalline or amorphous forms (i.e., as polymorphs). In general, all physical forms are equivalent for the uses contemplated by the present disclosure.

It will be apparent to one skilled in the art that certain compounds of the present disclosure may exist in tautomeric forms. Certain compounds of the present disclosure possess asymmetric carbon atoms (optical centers) or double bonds; the racemates, diastereomers, geometric isomers and individual isomers (e.g., separate enantiomers). The compounds of the present disclosure may also contain unnatural proportions of atomic isotopes at one or more of the atoms that constitute such compounds. Unnatural proportions of an isotope may be defined as ranging from the amount found in nature to an amount consisting of 100% of the atom in question. For example, the compounds may incorporate radioactive isotopes, such as for example tritium (³H), iodine-125 (¹²⁵I) or carbon-14 (¹⁴C), or non-radioactive isotopes, such as deuterium (²H) or carbon-13 (¹³C). Such isotopic variations can provide additional utilities to those described elsewhere within this application. For instance, isotopic variants of the compounds of the invention may find additional utility as diagnostic and/or imaging reagents, or as cytotoxic/radiotoxic therapeutic agents. Additionally, isotopic variants of the compounds of the invention can have altered pharmacokinetic and pharmacodynamic characteristics which can contribute to enhanced safety, tolerability or efficacy during treatment.

### Compounds that Modulate CCR2 Activity

The present disclosure provides compounds that modulate CCR2 activity. Chemokine receptors are integral membrane proteins which interact with an extracellular ligand, such as a chemokine, and mediate a cellular response to the ligand, e.g., chemotaxis, increased intracellular calcium ion concentration, etc. Therefore, modulation of a chemokine receptor function, e.g., interference with a chemokine receptor ligand interaction, will modulate a chemokine receptor mediated response, and treat or prevent a chemokine receptor mediated condition or disease. Modulation of a chemokine receptor function includes both inducement and inhibition of the function. The type of modulation accomplished will depend on the characteristics of the compound, i.e., antagonist or full, partial or inverse agonist.

Without intending to be bound by any particular theory, it is believed that the compounds provided herein interfere with the interaction between a chemokine receptor and one or more cognate ligands. In particular, it is believed that the compounds interfere with the interaction between CCR2 and a CCR2 ligand, such as MCP-1. Compounds contemplated by the disclosure include the exemplary compounds provided herein and salts thereof.

The compounds of the disclosure are thought to interfere with inappropriate T-cell trafficking by specifically modulating or inhibiting a chemokine receptor function.. Compounds contemplated by the disclosure include the exemplary compounds provided herein and salts thereof.

### Compounds

One embodiment of the present invention is the compound of Formula I, or salts thereof, for use in treating pancreatic cancer or in controlling an adenocarcinoma: 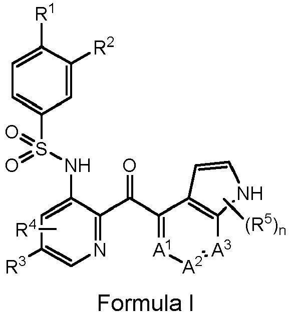
R¹ is halogen or C₁₋₆ alkyl;
R² is hydrogen, halogen, C₁₋₆ alkyl, C₁₋₆ alkoxy, C₁₋₆ haloalkyl, C₁₋₆ haloalkoxy, or - CN;
R³ is hydrogen, halogen or C₁₋₆ alkyl;
R⁴ is hydrogen, halogen, or C₁₋₆ alkyl;
each R⁵ if present is independently C₁₋₆ alkyl, -OH, or -NH₂;
n is 0, 1, 2, or 3; and
each of A¹, A² and A³ is -CH- or -N-, where at least one of A¹, A² or A³ is -N-.

In as much as any composition described herein defines any atom as nitrogen, i.e. for A¹, A² and A³, the person of ordinary skill in the art should understand that the nitrogen atom would maintain its aromaticity.

In one embodiment, R¹ is halogen or methyl; R2 is halogen or C₁₋₆ haloalkyl; R³ is halogen or C₁₋₆ alkyl; R⁴ is hydrogen; n is 0; A¹ is -CH- or -N; A² is -CH; and A³ is -N-. In one embodiment, R¹ is halogen or methyl; R² is C₁₋₃ haloalkyl; R³ is halogen or C₁₋₃ alkyl; R⁴ is hydrogen; n is 0; A¹ is -CH- or -N-; A² is -CH-; and A³ is -N-. In one embodiment, the compound is selected from the following formula, or salts thereof: 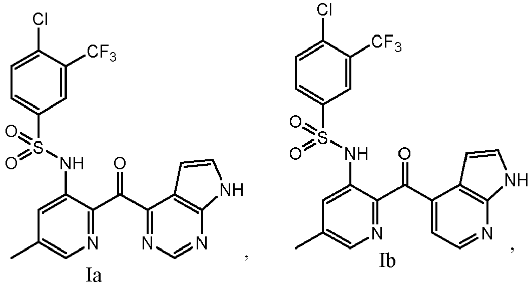 and

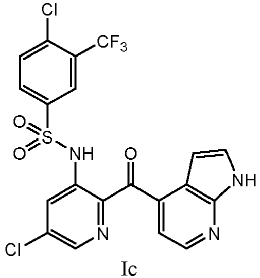

In another embodiment, the compound is the compound of Formula Ib, or salts thereof:

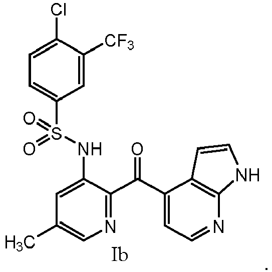

### Compositions

Pharmaceutically acceptable compositions can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compound(s), a liquid dosage form may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid may be incorporated in an injectable product. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the disclosure, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this disclosure with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar--agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols.

The compounds of the present disclosure or a pharmaceutically acceptable salt thereof may be formulated using nanotechnology. Nanoparticles are attractive for medical purposes based on their unique features, such as their surface to mass ratio being larger than that of other particles, their quantum properties, and their ability to adsorb and carry other compounds. Nanoparticles may have dimensions below 0.1 µm or 100 nm. Alternatively, a pharmaceutical composition may comprise relatively large (size >100 nm) nanoparticles, as needed for loading a sufficient amount of drug onto the particles. In addition, for drug delivery, not only engineered particles may be used as carrier, but also the drug itself may be formulated at a nanoscale, and then function as its own carrier. The composition of the engineered nanoparticles may vary. Source materials may be of biological origin like phospholipids, lipids, lactic acid, dextran, chitosan, or have more chemical characteristics like various polymers, carbon, silica, and metals. Especially in the area of engineered nanoparticles of polymer origin there is a vast area of possibilities for the chemical composition. See, for example, Martins et al., Nanoparticle Drug Delivery Systems: Recent Patents and Applications in Nanomedicine, Recent Patents on Nanomedicine, 2013, 3(2), pp 1 - 14.

The compounds of the present disclosure or a pharmaceutically acceptable salt thereof may also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this disclosure include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated. Additionally, the disclosure contemplates the use of transdermal patches, which have the added advantage of providing controlled delivery of a compound to the body. Such dosage forms are prepared by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

### Use in Methods of Treatment

The compound of Formula I is useful in methods for treating pancreatic cancer in a patient or in controlling an adenocarcinoma comprising administrating to the patient in need thereof an effective amount of any of the compounds of Forumla I described herein. In one embodiment, the pancreatic cancer is Stage I, II, III or IV.

In one embodiment, the treatment provides one or more of a decrease in tumor size, a suppression or decrease in tumor growth, no new tumor formation, a decrease in new tumor formation, an increase in survival or progression-free survival, no metastases, an increase in treatment options, delay in time from surgery to recurrence, reduction in jaundice, suppression of spread to liver, reduction in pain, improved appetite, improved digestion, reduction of gallbladder size, and reduced incidence of blood clots. In one embodiment, the patient achieves a complete response. In one embodiment, the patient achieves a partial response. In one embodiment, the patient achieves stable disease. In one embodiment, the patient achieves a slower progressive disease.

In one embodiment, the patient suffers from one or more of pancreatic adenocarcinoma, non-resectable pancreatic cancer, locally advanced pancreatic cancer, borderline resectable pancreatic cancer, locally advanced pancreatic ductal adenocarcinoma, borderline resectable pancreatic ductal adenocarcinoma, metastatic pancreatic cancer, chemotherapy-resistant pancreatic cancer, pancreatic ductal adenocarcinoma, squamous pancreatic cancer, pancreatic progenitor, immunogenic pancreatic cancer, aberrantly differentiated endocrine exocrine (ADEX) tumors, an exocrine pancreatic cancer, pancreatic intraepithelial neoplasia, intraductal papillary mucinous neoplasms, mucinous cystic neoplasms, mucinous pancreas cancer, adenosquamous carcinoma, signet ring cell carcinoma, hepatoid carcinoma, colloid carcinoma, undifferentiated carcinoma, undifferentiated carcinomas with osteoclast-like giant cells, a pancreatic cystic neoplasm, an islet cell tumor, a pancreas endrocrine tumor, or a pancreatic neuroendrocrine tumor.

In one embodiment, the compound of Formula I is provided as a pharmaceutical composition for oral administration. In one embodiment, the compound is administered once a day. In one embodiment, the compound is administered twice a day.

In one embodiment, the treatment further comprises administering to the patient one or more additional therapeutic compound. In one embodiment, the one or more additional therapeutic compound is selected from one or more of a Btk tyrosine kinase inhibitor, an Erbb2 tyrosine kinase receptor inhibitor; an Erbb4 tyrosine kinase receptor inhibitor, an mTOR inhibitor, a thymidylate synthase inhibitor, an EGFR tyrosine kinase receptor inhibitor, an epidermal growth factor antagonist, a Fyn tyrosine kinase inhibitor, a kit tyrosine kinase inhibitor, a Lyn tyrosine kinase inhibitor, a NK cell receptor modulator, a PDGF receptor antagonist, a PARP inhibitor, a poly ADP ribose polymerase inhibitor, a poly ADP ribose polymerase 1 inhibitor, a poly ADP ribose polymerase 2 inhibitor, a poly ADP ribose polymerase 3 inhibitor, a galactosyltransferase modulator, a dihydropyrimidine dehydrogenase inhibitor, an orotate phosphoribosyltransferase inhibitor, a telomerase modulator, a mucin 1 inhibitor, a mucin inhibitor, a secretin agonist, a TNF related apoptosis inducing ligand modulator, an IL17 gene stimulator, an interleukin 17E ligand, a Neurokinin receptor agonist, a cyclin G1 inhibitor, a checkpoint inhibitor, a PD-1 inhibitor, a PD-L1 inhibitor, a CTLA4 inhibitor, a topoisomerase I inhibitor, an Alk-5 protein kinase inhibitor, a connective tissue growth factor ligand inhibitor, a notch-2 receptor antagonist, a notch-3 receptor antagonist, a hyaluronidase stimulator, a MEK-1 protein kinase inhibitor; MEK-2 protein kinase inhibitor, a GM-CSF receptor modulator; TNF alpha ligand modulator, a mesothelin modulator, an asparaginase stimulator, a caspase-3 stimulator; caspase-9 stimulator, a PKN3 gene inhibitor, a hedgehog protein inhibitor; Smoothened receptor antagonist, an AKT1 gene inhibitor, a DHFR inhibitor, a thymidine kinase stimulator, a CD29 modulator, a fibronectin modulator, an interleukin-2 ligand, a serine protease inhibitor, a D40LG gene stimulator; TNFSF9 gene stimulator, a 2-oxoglutarate dehydrogenase inhibitor, a TGF-beta type II receptor antagonist, an Erbb3 tyrosine kinase receptor inhibitor, a cholecystokinin CCK2 receptor antagonist, a Wilms tumor protein modulator, a Ras GTPase modulator, an histone deacetylase inhibitor, a cyclin-dependent kinase 4 inhibitor A modulator, an estrogen receptor beta modulator, a 4-1BB inhibitor, a 4-1BBL inhibitor, a PD-L2 inhibitor, a B7-H3 inhibitor, a B7-H4 inhibitor, a BTLA inhibitor, a HVEM inhibitor, aTIM3 inhibitor, a GAL9 inhibitor, a LAG3 inhibitor, a VISTA inhibitor, a KIR inhibitor, a 2B4 inhibitor, a CD160 inhibitor and a CD66e modulator. In one embodiment, the one or more additional therapeutic compound is selected from one or more of bavituximab, IMM-101, CAP1-6D, Rexin-G , genistein, CVac, MM-D37K, PCI-27483, TG-01, mocetinostat, LOAd-703, CPI-613, upamostat, CRS-207, NovaCaps, trametinib, Atu-027, sonidegib, GRASPA, trabedersen, nastorazepide, Vaccell, oregovomab, istiratumab, refametinib, regorafenib, lapatinib, selumetinib, rucaparib, pelareorep, tarextumab, PEGylated hyaluronidase, varlitinib, aglatimagene besadenovec, GBS-01, GI-4000, WF-10, galunisertib, afatinib, RX-0201, FG-3019, pertuzumab, DCVax-Direct, selinexor, glufosfamide, virulizin, yttrium (90Y) clivatuzumab tetraxetan, brivudine, nimotuzumab, algenpantucel-L, tegafur + gimeracil + oteracil potassium + calcium folinate, olaparib, ibrutinib, pirarubicin, Rh-Apo2L, tertomotide, tegafur + gimeracil + oteracil potassium, tegafur + gimeracil + oteracil potassium, masitinib, Rexin-G, mitomycin, erlotinib , adriamycin, dexamethasone, vincristine, cyclophosphamide, fluorouracil, topotecan, taxol, interferons, platinum derivatives, taxane, paclitaxel, vinca alkaloids, vinblastine, anthracyclines, doxorubicin, epipodophyllotoxins, etoposide, cisplatin, rapamycin, methotrexate, actinomycin D, dolastatin 10, colchicine, emetine, trimetrexate, metoprine, cyclosporine, daunorubicin, teniposide, amphotericin, alkylating agents, chlorambucil, 5-fluorouracil, campthothecin, metronidazole, Gleevec, Avastin, Vectibix, abarelix, aldesleukin, alemtuzumab, alitretinoin, allopurinol, altretamine, amifostine, anastrozole, arsenic trioxide, asparaginase, azacitidine, AZD9291, BCG Live, bevacuzimab, fluorouracil, bexarotene, bleomycin, bortezomib, busulfan, calusterone, capecitabine, camptothecin, carboplatin, carmustine, celecoxib, cetuximab, chlorambucil, cladribine, clofarabine, cyclophosphamide, cytarabine, dactinomycin, darbepoetin alfa, daunorubicin, denileukin, dexrazoxane, docetaxel, doxorubicin (neutral), doxorubicin hydrochloride, dromostanolone propionate, epirubicin, epoetin alfa, estramustine, etoposide phosphate, etoposide, exemestane, filgrastim, floxuridine fludarabine, fulvestrant, gefitinib, gemcitabine, gemtuzumab, goserelin acetate, histrelin acetate, hydroxyurea, ibritumomab, idarubicin, ifosfamide, imatinib mesylate, interferon alfa-2a, interferon alfa-2b, irinotecan, lenalidomide, letrozole, leucovorin, leuprolide acetate, levamisole, lomustine, megestrol acetate, melphalan, mercaptopurine, 6-MP, mesna, methotrexate, methoxsalen, mitomycin C, mitotane, mitoxantrone, nandrolone, nelarabine, nofetumomab, oprelvekin, oxaliplatin, paclitaxel, palifermin, pamidronate, pegademase, pegaspargase, pegfilgrastim, pemetrexed disodium, pentostatin, pipobroman, plicamycin, porfimer sodium, procarbazine, quinacrine, rasburicase, rituximab, rociletinib, sargramostim, sorafenib, streptozocin, sunitinib maleate, talc, tamoxifen, temozolomide, teniposide, VM-26, testolactone, thioguanine, 6-TG, thiotepa, topotecan, toremifene, tositumomab, trastuzumab, tretinoin, ATRA, uracil mustard, valrubicin, vinblastine, vincristine, vinorelbine, zoledronate, zoledronic acid, pembrolizumab, nivolumab, IBI-308, mDX-400, BGB-108, MEDI-0680, SHR-1210, PF-06801591, PDR-001, GB-226, STI-1110, durvalumab, atezolizumab, avelumab, BMS-936559, ALN-PDL, TSR-042, KD-033, CA-170, STI-1014, FOLFIRINOX and KY-1003. In one embodiment, the one or more additional therapeutic compound is FOLFIRINOX. In one embodiment, the one or more additional therapeutic compounds are gemcitabine and paclitaxel. In one embodiment, the one or more additional therapeutic compounds are gemcitabine and nab-paclitaxel.

In one embodiment the compounf of Formula I is a compound of Formula Ib:

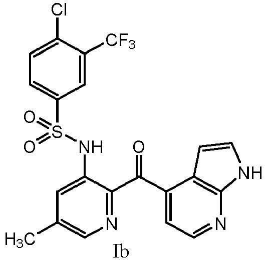

In one embodiment, the effective amount is from 50 mg to 300 mg. In one embodiment, the effective amount is 150 mg. One embodiment of the present invention is a pharmaceutical combination comprising the compound of Formula I: 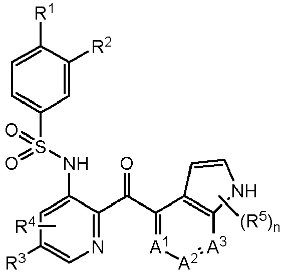 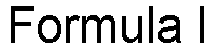 or a pharmaceutically acceptable salt thereof, wherein R¹ is halogen or C₁₋₆ alkyl; R² is hydrogen, halogen, C₁₋₆ alkyl, C₁₋₆ alkoxy, C₁₋₆ haloalkyl, C₁₋₆ haloalkoxy, or -CN; R³ is hydrogen, halogen, or C₁₋₆ alkyl; R⁴ is hydrogen, halogen, or C₁₋₆ alkyl; each R⁵ is independently C₁₋₆ alkyl, -OH, or -NH₂; n is 0, 1, 2, or 3; each of A¹, A², and A³ is -CH- or - N-, where at least one of A¹, A², or A³ is -N-; and one or more additional therapeutic compound. In one embodiment, the one or more additional therapeutic compound is selected from one or more of bavituximab, IMM-101, CAP1-6D, Rexin-G , genistein, CVac, MM-D37K, PCI-27483, TG-01, mocetinostat, LOAd-703, CPI-613, upamostat, CRS-207, NovaCaps, trametinib, Atu-027, sonidegib, GRASPA, trabedersen, nastorazepide, Vaccell , oregovomab, istiratumab, refametinib, regorafenib, lapatinib, selumetinib, rucaparib, pelareorep, tarextumab, PEGylated hyaluronidase, varlitinib, aglatimagene besadenovec, GBS-01, GI-4000, WF-10, galunisertib, afatinib, RX-0201, FG-3019, pertuzumab, DCVax-Direct, selinexor, glufosfamide, virulizin, yttrium (90Y) clivatuzumab tetraxetan, brivudine, nimotuzumab, algenpantucel-L, tegafur + gimeracil + oteracil potassium + calcium folinate, olaparib, ibrutinib, pirarubicin, Rh-Apo2L, tertomotide, tegafur + gimeracil + oteracil potassium, tegafur + gimeracil + oteracil potassium, masitinib, Rexin-G, mitomycin, erlotinib , adriamycin, dexamethasone, vincristine, cyclophosphamide, fluorouracil, topotecan, taxol, interferons, platinum derivatives, taxane, paclitaxel, vinca alkaloids, vinblastine, anthracyclines, doxorubicin, epipodophyllotoxins, etoposide, cisplatin, rapamycin, methotrexate, actinomycin D, dolastatin 10, colchicine, emetine, trimetrexate, metoprine, cyclosporine, daunorubicin, teniposide, amphotericin, alkylating agents, chlorambucil, 5-fluorouracil, campthothecin, cisplatin, metronidazole, Gleevec, Avastin, Vectibix, abarelix, aldesleukin, alemtuzumab, alitretinoin, allopurinol, altretamine, amifostine, anastrozole, arsenic trioxide, asparaginase, azacitidine, AZD9291, BCG Live, bevacuzimab, fluorouracil, bexarotene, bleomycin, bortezomib, busulfan, calusterone, capecitabine, camptothecin, carboplatin, carmustine, celecoxib, cetuximab, chlorambucil, cladribine, clofarabine, cyclophosphamide, cytarabine, dactinomycin, darbepoetin alfa, daunorubicin, denileukin, dexrazoxane, docetaxel, doxorubicin (neutral), doxorubicin hydrochloride, dromostanolone propionate, epirubicin, epoetin alfa, estramustine, etoposide phosphate, etoposide, exemestane, filgrastim, floxuridine fludarabine, fulvestrant, gefitinib, gemcitabine, gemtuzumab, goserelin acetate, histrelin acetate, hydroxyurea, ibritumomab, idarubicin, ifosfamide, imatinib mesylate, interferon alfa-2a, interferon alfa-2b, irinotecan, lenalidomide, letrozole, leucovorin, leuprolide acetate, levamisole, lomustine, megestrol acetate, melphalan, mercaptopurine, 6-MP, mesna, methotrexate, methoxsalen, mitomycin C, mitotane, mitoxantrone, nandrolone, nelarabine, nofetumomab, oprelvekin, oxaliplatin, paclitaxel, palifermin, pamidronate, pegademase, pegaspargase, pegfilgrastim, pemetrexed disodium, pentostatin, pipobroman, plicamycin, porfimer sodium, procarbazine, quinacrine, rasburicase, rituximab, rociletinib, sargramostim, sorafenib, streptozocin, sunitinib maleate, talc, tamoxifen, temozolomide, teniposide, VM-26, testolactone, thioguanine, 6-TG, thiotepa, topotecan, toremifene, tositumomab, trastuzumab, tretinoin, ATRA, uracil mustard, valrubicin, vinblastine, vincristine, vinorelbine, zoledronate, zoledronic acid, pembrolizumab, nivolumab, IBI-308, mDX-400, BGB-108, MEDI-0680, SHR-1210, PF-06801591, PDR-001, GB-226, STI-1110, durvalumab, atezolizumab, avelumab, BMS-936559, ALN-PDL, TSR-042, KD-033, CA-170, STI-1014, FOLFIRINOX and KY-1003. In one embodiment, the one or more additional therapeutic compound is FOLFIRINOX. In one embodiment, the one or more additional therapeutic compounds are gemcitabine and paclitaxel. In one embodiment, the one or more additional therapeutic compounds are gemcitabine and nab-paclitaxel.

In one embodiment, the combination comprises a fixed dose combination or separate doses.

In one embodiment, R¹ is halogen or methyl; R² is halogen or C₁₋₆ haloalkyl; R³ is halogen or C₁₋₆ alkyl; R⁴ is hydrogen; n is 0; A¹ is -CH- or-N-; A² is -CH-; and A³ is -N-. In one embodiment, R¹ is halogen or methyl; R² is C₁₋₃ haloalkyl; R³ is halogen or C₁₋₃ alkyl; R⁴ is hydrogen; n is 0; A¹ is -CH- or -N-; A² is -CH-; and A³ is -N-.

In one embodiment, the compound is selected from the following formulas or salts thereof: 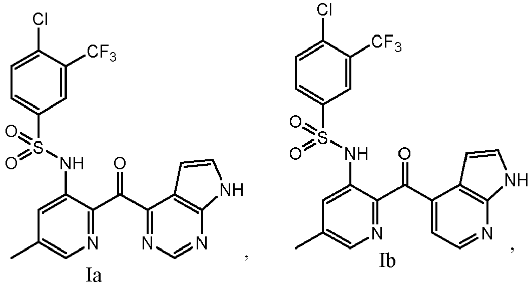 and

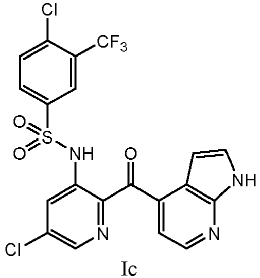

In one embodiment, the compound is the compound of Formula Ib:

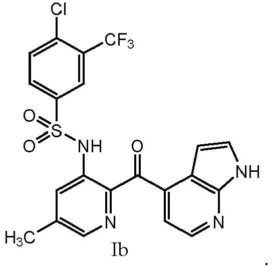

In one embodiment, treating comprises administration of the pharmaceutical combination of any one of compounds described herein.

A method of limiting over-expression of oncogenes, activating tumor suppressor genes, or regulating signaling proteins may comprise administering to a patient in need thereof an effective amount of the compound of Formula I as described above: 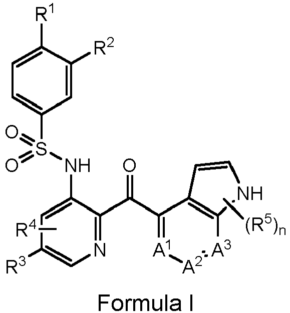 or a pharmaceutically acceptable salt thereof. The compound may be selected from 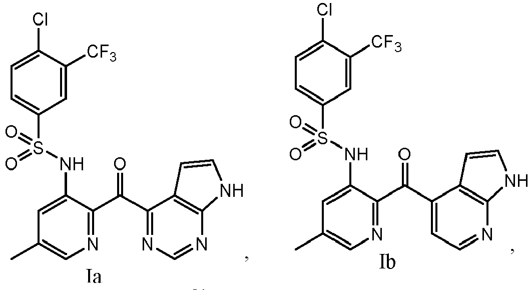 and 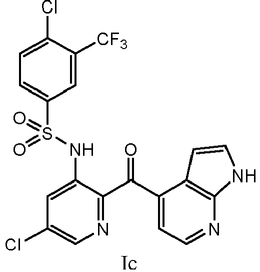 or a pharmaceutically acceptable salt thereof. The compound may be the compound of Formula Ib: 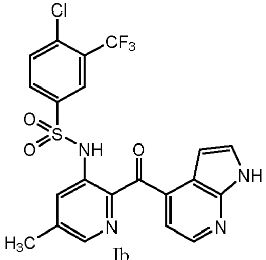 or a pharmaceutically acceptable salt thereof.

The over-expression of oncogenes, inactivation tumor suppressor genes or the deregulation of various signaling proteins can result in diagnosis of pancreatic cancer for the patient. The compound of Formula I may be provided as a pharmaceutical composition for oral administration. The treatment may further comprise administering to the patient one or more additional therapeutic compound as defined above.

### Dosing regimen

Provided herein is a dosing regimen for treating pancreatic cancer comprising administering a compound of Formula I, la, Ib or Ic for 6, 12, 24 or 48 weeks in combination 26 with Folfirinox or gemcitabine and paclitaxel or gemcitabine and nab paclitaxel. In some cases, the dosing regimen comprises administering a compound of Formula I, la, Ib or Ic for 12 weeks in combination with Folfirinox or gemcitabine and paclitaxel or gemcitabine and nab paclitaxel. In some cases, the dosing regimen comprises administering a compound of Formula Ib for 6, 12, 24 or 48 weeks in combination with Folfirinox or gemcitabine and paclitaxel or gemcitabine and nab paclitaxel. In some cases, the dosing regimen comprises administering a compound of Formula Ib for 12 weeks in combination with Folfirinox or gemcitabine and paclitaxel or gemcitabine and nab paclitaxel. In some cases, the dosing regimen comprises administering a compound of Formula Ib for 12 weeks in combination with Folfirinox.

Provided herein is a dosing regimen for treating pancreatic cancer comprising administering a compound of Formula I, la, Ib or Ic for 6, 12, 24 or 48 weeks in combination with Folfirinox or gemcitabine and paclitaxel or gemcitabine and nab paclitaxel followed by administration of fluorouracil (5-FU) alone or administration of the compound of Formula I, la, Ib or Ic alone. In some cases, the dosing regimen comprises administering a compound of Formula I, la, Ib or Ic for 12 weeks in combination with Folfirinox or gemcitabine and paclitaxel or gemcitabine and nab paclitaxel followed by administration of fluorouracil (5-FU) alone or administration of the compound of Formula I, la, Ib or Ic alone. In some cases, the dosing regimen comprises administering a compound of Formula Ib for 12 weeks in combination with Folfirinox or gemcitabine and paclitaxel or gemcitabine and nab paclitaxel followed by administration of fluorouracil (5-FU) alone or administration of the compound of Formula Ib alone. In some cases, the dosing regimen comprises administering a compound of Formula Ib for 12 weeks in combination with Folfirinox followed by administration of fluorouracil (5-FU) alone or administration of the compound of Formula Ib alone.

### EXAMPLES

### Example 1 - Study

Fifty-four patients age 18 years or older were enrolled on an open-label phase 1-b interventional study to evaluate the safety and efficacy of compound Ib in patients with pancreatic adenocarcinoma also receiving FOLFIRINOX chemotherapy. Dosing consisted of 150 mg tablets for oral administration once or twice daily given orally for at least 12 weeks.

Inclusion criteria included:
- Histologically or cytologically confirmed non-resectable pancreatic adenocarcinoma with or without metastases
- Eastern Cooperative Oncology Group (ECOG) performance status score ≤ 2
- Anticipated life expectancy ≥ 12 weeks
- Radiographically measurable disease acc. to RECIST 1.1
- Use of adequate contraception (as described in protocol)
- Ability to provide written informed consent and comply with study requirements.

Exclusion criteria included:
- Received other cancer treatment or investigational drug within 4 weeks prior to screening
- Women who are pregnant or breastfeeding
- Had major surgery within 4 weeks of first dose of study drug
- Inadequate liver, renal or bone marrow function within 2 weeks of first dose
- Serious concurrent illness, altered medical status or any uncontrolled medical condition
- Any infection requiring antibiotic or anti-viral treatment within 4 weeks of screening
- Known active HIV, HBV or HCV infection
- Inability to swallow tablets
- History or presence of any medical condition or disease which, in the opinion of the investigator, may place the subject at unacceptable risk for study participation.

### Example 2 - Pharmacokinetic parameters of compound Ib and PF-04136309 in Sprague-Dawley rats

Compound Ib and PF-04136309 were dosed intravenously at 0.5 mg/kg and orally at 2 mg/kg, in aqueous hydroxypropyl methylcellulose. Blood samples were collected at predetermined time points after each dosing and the corresponding plasma concentrations of Compound Ib and PF-04136309 were analyzed using an LC-MS/MS method. Plasma concentration-time curves were constructed and the corresponding pharmacokinetic parameters were derived using non-compartmental analysis.

Blood (0.2 mL) was sampled through the jugular vein or cardiac puncture (for terminal point only) at pre-dose, 2, 5, 10, 15, and 30 min, 1, 2, 4, 6, and 8 hours post-dose for i.v. dosing and at pre-dose, 5, 15, and 30 min, 1, 1.5, 2, 4, 6, and 8 hours post-dose for oral dosing. Blood samples were collected into chilled polypropylene tubes containing sodium EDTA as the anticoagulant and plasma was collected through centrifugation (Eppendorf Centrifuge 5417R) at 10,000 rpm and 4 °C for 6 minutes and stored at -20 °C until analysis.

Plasma samples (50 µL) were extracted with 200 µL acetonitrile containing the internal standard on a linear shaker for 10 min and then centrifuged at 3700g for 10 min at 4 °C (Allegra X-15R centrifuge, Beckman Coulter, Inc., Fullerton, CA). 100 µL of the resulting supernatant was transferred into a new plate and mixed with 100 µL 0.1% formic acid in water for LC-MS/MS analysis.

Calibration standard samples were prepared with blank Sprague-Dawley rat plasma, at 5000, 2500, 1000, 500, 100, 50, 20, 10, 4, 2 and 1 ng/mL of compound. Three levels of the standard stock solutions (1000, 100 and 10 ng/mL) were spiked separately into male Sprague-Dawley rat plasma and used as QC samples. Plasma standards and QC samples were treated identically and prepared in parallel with the plasma samples. Extracted samples were analyzed by LC-MS/MS.

Mass spectrometer acquisition and integration were performed with Applied Biosystems-Sciex Analyst software (version 1.4.2). The calibration curve was obtained through a quadratic regression and the calibration range was 2 - 5000 ng/mL.

Instrument:
- API 3000 mass spectrometer (Applied Biosystems, Foster City, CA)
- Agilent 1100 HPLC binary pump (Santa Clara, CA)
- LEAP Technologies HTS Pal Autosampler (Carrboro, NC)
- Thermo Scientific Cohesive Aria LX-2 duplexing system (Waltham, MA)
Column: Zorbax Eclipse XDB Phenyl 2.1×50 mm (Agilent, Santa Clara, CA)
Injection Volume: 10 µL
Flow Rate: 0.60 mL/min

**Table 1. HPLC Gradient**

| Time (min) | Mobile Phase A-0.1% formic acid in water | Mobile Phase B-0.1% formic acid in acetonitrile |
|---|---|---|
| 0 | 98 | 2 |
| 0.08 | 98 | 2 |
| 1.58 | 2 | 98 |
| 2.58 | 2 | 98 |
| 2.67 | 98 | 2 |
| 3.50 | 98 | 2 |

Ionization Mode: Electrospray (ESI). Detection Mode: Positive MRM. For each dosing route, descriptive pharmacokinetic parameters were determined by a standard non-compartmental analysis (Wagner, 1993) from the plasma concentration-time curve. Pharmacokinetic analysis was performed by using XLFit® v.4.3.1 (ID Business Solutions Inc., Alameda, CA) in conjunction with Microsoft Excel 2003.

**Table 2.**

| Pharmacokinetic parameters of compound Ib and PF-04136309 in Sprague-Dawley rats | | | |
|---|---|---|---|
| | | **Compound Ib** | **PF-04136309** |
| **Rat PK** | **Clearance** (iv, 0.5 mg/kg) | 11.5 mL/min/kg | 71.1 mL/min/kg |
| | **AUC** (po, 2 mg/kg) | 3,910 ng*hr/mL | 111 ng*hr/mL |
| | **Oral Bioavailability** | 133% | 22% |

Compared to PF-4136309, compound Ib has a much lower clearance and much higher exposure (AUC).

### Example 3 - Pharmacokinetic parameters of compound Ib in beagle dogs

The pharmacokinetic profile of compound Ib was evaluated in male beagle dogs following intravenous (i.v.) and oral (p.o.) administration. A single dose of compound Ib was dosed intravenously at 0.5 mg/kg and orally at 2 mg/kg. Blood samples were collected at predetermined time points after each dosing and the corresponding plasma samples were analyzed using an LC-MS/MS method. Plasma concentration-time curves were constructed using the plasma concentrations and the corresponding pharmacokinetic parameters were derived by non-compartmental analysis.

Six animals weighing 11.5, 9.9, 9.9, 14.6, 11.1, and 9.8 kg, respectively, were used with Compound Ib dosed intravenously at 0.5 mg/kg (the first 3 animals) and orally at 2 mg/kg (the second 3 animals).

For i.v. dosing, a solution formulation of Compound Ib was prepared in 36.8% water / 31.6% propylene glycol / 31.6% N,N-dimethyl acetamide at 0.5 mg/mL and each animal received 1 mL/kg. For oral dosing at 2 mg/kg, a solution formulation was prepared in 1% hydroxypropyl methylcellulose at 0.5 mg/mL and each animal received 4 mL/kg.

For dosing, blood (∼1 mL) was drawn at pre-dose and at 5, 15, and 30 min, 1, 2, 4, 8, 12, and 24 hours post-dose. Blood was sampled from the foreleg vein via butterfly catheter and then placed into chilled polypropylene tubes containing K2EDTA as the anticoagulant and kept on ice until centrifugation. Plasma was collected through centrifugation and shipped on dry ice for sample analysis.

Plasma samples (50 µL) were extracted with 200 µL 0.1% formic acid/acetonitrile containing the internal standard on a linear shaker for 10 min and then centrifuged at 3700g for 10 min at 4 °C (Allegra X-15R centrifuge, Beckman Coulter, Inc., Fullerton, CA). 100 µL of the resulting supernatant was transferred into a new plate and mixed with 100 µL 0.1% formic acid in water for LC-MS/MS analysis.

Calibration standard samples were prepared with blank dog plasma, at 5000, 2500, 1000, 500, 100, 50, 20, 10, 4, 2 and 1 ng/mL of Compound lb. Three levels of the standard stock solutions (1000, 100 and 10 ng/mL) were spiked separately into male beagle dog plasma and used as QC samples. Plasma standards and QC samples were treated identically and prepared in parallel with the plasma samples.

Sample analysis was performed by LC-MS/MS (details shown below). Acquisition and peak integration were performed with the Applied Biosystems-Sciex Analyst software (version 1.4.2). Calibration curves were obtained through quadratic regression with a weighting of 1/x. The calibration range was 1 - 5000 ng/mL.

Instrument:
- Applied Biosystems API 3000 mass spectrometer (Foster City, CA)
- Agilent 1100 HPLC binary pump (Santa Clara, CA)
- LEAP Technologies HTS Pal Autosampler (Carrboro, NC)
- Thermo Scientific Cohesive Aria LX-2 duplexing system (Waltham, MA)
Column: Zorbax Eclipse XDB Phenyl 2.1x50 mm (Agilent, Santa Clara, CA)
Injection Volume: 10 µL
Flow Rate: 0.60 mL/min

**Table 3: HPLC Gradient**

| **Time (min)** | **Mobile Phase A** 0.1% formic acid in water | **Mobile Phase B** 0.1% formic acid in acetonitrile |
|---|---|---|
| 0 | 98 | 2 |
| 0.08 | 98 | 2 |
| 1.58 | 2 | 98 |
| 2.58 | 2 | 98 |
| 2.67 | 98 | 2 |
| 3.50 | 98 | 2 |

Ionization Mode: Turbo lonspray ESI
Detection Mode: Positive MRM

For each dosing route, descriptive pharmacokinetic parameters were determined by a standard non-compartmental analysis (Wagner, 1993) from the plasma concentration-time curve.
- CL: Total body clearance
- AUC: area under the curve
- F: Bioavailability.

Pharmacokinetic analysis was performed by using XLFit® v.4.3.1 (ID Business Solutions Inc., Alameda, CA) in conjunction with Microsoft Excel 2003.

**Table 4: Mean pharmacokinetic parameters of Compound Ib and PF-04136309 following i.v. and oral dosings of Ib (N=3)**

| | | **Compound Ib** | **PF-04136309 (data extracted from ACS Med. Chem. Lett. reference)** |
|---|---|---|---|
| **Dog PK** | **Clearance** iv | 0.7 (dosed at 0.5 mg/kg) | 7.6 (dosed at 2 mg/kg) |
| | **AUC** po | 67000 ng*hr/mL (dosed at 2 mg/kg) | 20198 ng*hr/mL (dosed at 10mg/kg) |
| | **Oral Bioavailability** | 143% (dosed at 2 mg/kg) | 78% (dosed at 10mg/kg) |

The pharmacokinetics parameters of PF-4136309 were disclosed in ACS Med. Chem. Lett. 2011, 2, 913-918.

Compared to PF-4136309, compound Ib has a much lower clearance and much higher exposure (AUC) at comparable doses.

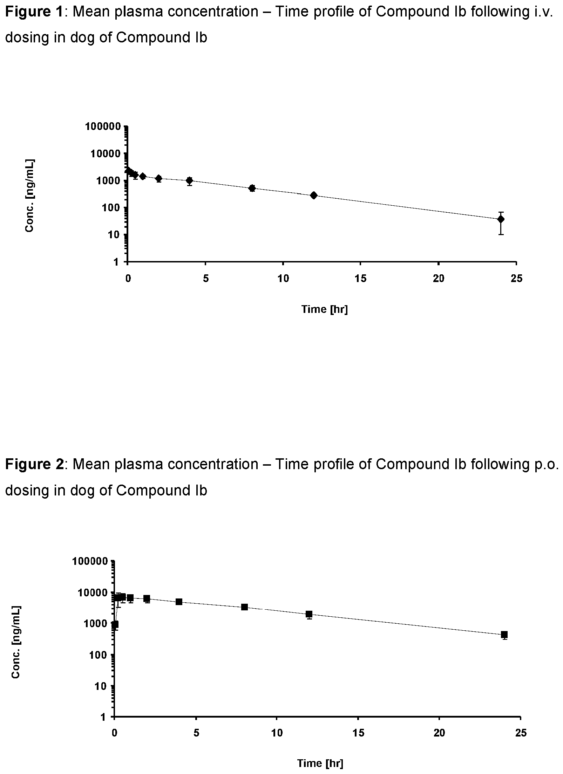

Figure 1 represents the mean plasma concentration of Compound Ib following i.v. dosing in dog of Compound lb.

Figure 2 represents the mean plasma concentration of Compound Ib following p.o. dosing in dog of Compound lb.

The specific pharmacological responses observed may vary according to and depending on the particular active compound selected or whether there are present pharmaceutical carriers, as well as the type of formulation and mode of administration employed, and such expected variations or differences in the results are contemplated in accordance with practice of the present disclosure.

## Claims

1. A compound of Formula I: 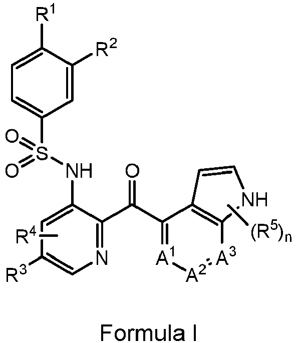 or a pharmaceutically acceptable salt thereof,
wherein
R¹ is halogen or C₁-₆ alkyl;
R² is hydrogen, halogen, C₁₋₆ alkyl, C₁₋₆ alkoxy, C₁₋₆ haloalkyl, C₁₋₆ haloalkoxy, or -CN;
R³ is hydrogen, halogen, or C₁₋₆ alkyl;
R⁴ is hydrogen, halogen, or C₁₋₆ alkyl;
each R⁵ is independently C₁₋₆ alkyl, -OH, or -NH₂;
n is 0, 1, 2, or 3; and
each of A¹, A², and A³ is -CH- or -N-, where at least one of A¹, A², or A³ is -N-for use in treating pancreatic cancer or in controlling an adenocarcinoma.

2. The compound for use of claim 1 wherein
R¹ is halogen or methyl;
R² is halogen or C₁₋₆ haloalkyl;
R³ is halogen or C₁₋₆ alkyl;
R⁴ is hydrogen;
n is 0;
A¹ is -CH- or -N-;
A² is -CH-; and
A³ is -N-.

3. The compound for use of claim 1, wherein the compound is selected from the group consisting of: 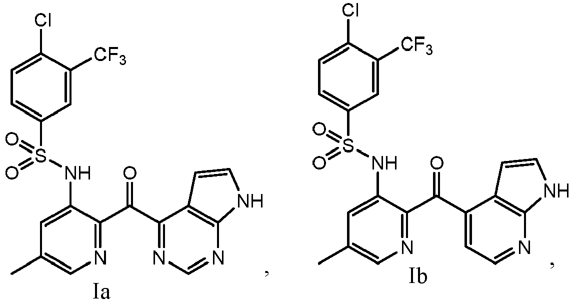 and 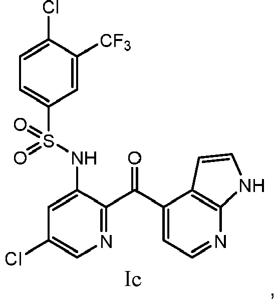 or a pharmaceutically acceptable salt thereof.

4. The compound for use of claim 1, wherein the compound is 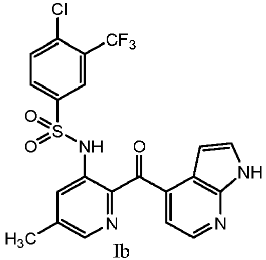 or a pharmaceutically acceptable salt thereof.

5. The compound for use of any one of claims 1 to 4, for controlling a pancreatic adenocarcinoma.

6. The compound for use of any one of claims 1 to 5, wherein the compound of Formula I is provided as a pharmaceutical composition for oral administration.

7. The compound for use of any one of claims 1 to 6, wherein the effective amount is from 50 mg to 300 mg, preferably 150 mg.

8. The compound for use of any one of claims 1 to 7, wherein the compound is administered once a day or twice a day.

9. The compound for use of any one of claims 1 to 8, the use further comprising administering to the patient one or more additional therapeutic compound, wherein the one or more additional therapeutic compound is selected from one or more of a Btk tyrosine kinase inhibitor, an Erbb2 tyrosine kinase receptor inhibitor; an Erbb4 tyrosine kinase receptor inhibitor, an mTOR inhibitor, a thymidylate synthase inhibitor, an EGFR tyrosine kinase receptor inhibitor, an Epidermal growth factor antagonist, a Fyn tyrosine kinase inhibitor, a kit tyrosine kinase inhibitor, a Lyn tyrosine kinase inhibitor, a NK cell receptor modulator, a PDGF receptor antagonist, a PARP inhibitor, a poly ADP ribose polymerase inhibitor, a poly ADP ribose polymerase 1 inhibitor, a poly ADP ribose polymerase 2 inhibitor, a poly ADP ribose polymerase 3 inhibitor, a galactosyltransferase modulator, a dihydropyrimidine dehydrogenase inhibitor, an orotate phosphoribosyltransferase inhibitor, a telomerase modulator, a mucin 1 inhibitor, a mucin inhibitor, a secretin agonist, a TNF related apoptosis inducing ligand modulator, an IL17 gene stimulator, an interleukin 17E ligand, a Neurokinin receptor agonist, a cyclin G1 inhibitor, a checkpoint inhibitor, a PD-1 inhibitor, a PD-L1 inhibitor, a CTLA4 inhibitor, a topoisomerase I inhibitor, an Alk-5 protein kinase inhibitor, a connective tissue growth factor ligand inhibitor, a notch-2 receptor antagonist, a notch-3 receptor antagonist, a hyaluronidase stimulator, a MEK-1 protein kinase inhibitor; MEK-2 protein kinase inhibitor, a GM-CSF receptor modulator; TNF alpha ligand modulator, a mesothelin modulator, an asparaginase stimulator, a caspase-3 stimulator; caspase-9 stimulator, a PKN3 gene inhibitor, a hedgehog protein inhibitor; Smoothened receptor antagonist, an AKT1 gene inhibitor, a DHFR inhibitor, a thymidine kinase stimulator, a CD29 modulator, a fibronectin modulator, an interleukin-2 ligand, a serine protease inhibitor, a D40LG gene stimulator; TNFSF9 gene stimulator, a 2-oxoglutarate dehydrogenase inhibitor, a TGF-beta type II receptor antagonist, an Erbb3 tyrosine kinase receptor inhibitor, a cholecystokinin CCK2 receptor antagonist, a Wilms tumor protein modulator, a Ras GTPase modulator, an histone deacetylase inhibitor, a cyclin-dependent kinase 4 inhibitor A modulator, an estrogen receptor beta modulator, a 4-1BB inhibitor, a 4-1BBL inhibitor, a PD-L2 inhibitor, a B7-H3 inhibitor, a B7-H4 inhibitor, a BTLA inhibitor, a HVEM inhibitor, aTIM3 inhibitor, a GAL9 inhibitor, a LAG3 inhibitor, a VISTA inhibitor, a KIR inhibitor, a 2B4 inhibitor, a CD160 inhibitor and a CD66e modulator.

10. The compound for use of claim 9, wherein the one or more additional therapeutic compound is FOLFIRINOX, or wherein the one or more additional therapeutic compounds are gemcitabine and paclitaxel.

11. A pharmaceutical composition for use in treating pancreatic cancer or in controlling an adenocarcinoma comprising a compound as defined in any one of claims 1 to 5 and a pharmaceutically acceptable excipient.

12. The pharmaceutical composition for use of claim 11 comprising one or more additional therapeutic compound in addition to the compound as defined in any one of claims 1 to 5, wherein the one or more additional therapeutic compound is FOLFIRINOX, or wherein the one or more additional therapeutic compounds are gemcitabine and paclitaxel.

## Patentansprüche

1. Eine Verbindung der Formel I: 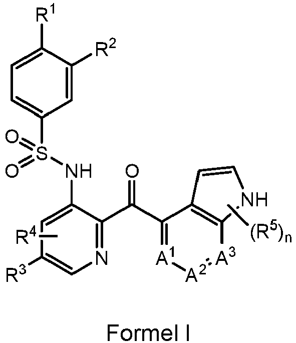 oder ein pharmazeutisch akzeptables Salz davon,
worin
R¹ Halogen oder C₁₋₆-Alkyl ist;
R² Wasserstoff, Halogen, C₁₋₆-Alkyl, C₁₋₆-Alkoxy, C₁₋₆-Haloalkyl, C₁₋₆-Haloalkoxy, oder -CN ist;
R³ Wasserstoff, Halogen, oder C₁₋₆-Alkyl ist;
R⁴ Wasserstoff, Halogen, oder C₁₋₆-Alkyl ist;
jedes R⁵ unabhängig voneinander C₁₋₆-Alkyl, -OH, oder -NH₂ ist;
n 0, 1, 2, oder 3 ist; und
jedes von A¹, A², und A³-CH- oder -N- ist, wobei mindestens eines von A¹, A², oder A³ -N- ist,
zur Verwendung in der Behandlung von Bauchspeicheldrüsenkrebs oder bei der Bekämpfung eines Adenokarzinoms.

2. Die Verbindung zur Verwendung gemäß Anspruch 1, worin
R¹ Halogen oder Methyl ist;
R² Halogen oder C₁₋₆-Haloalkyl ist;
R³ Halogen oder C₁₋₆-Alkyl ist;
R⁴ Wasserstoff ist;
n 0 ist;
A¹ -CH- oder -N- ist;
A² -CH- ist; und
A³ -N- ist.

3. Die Verbindung zur Verwendung gemäß Anspruch 1, wobei die Verbindung ausgewählt ist aus der Gruppe bestehend aus: 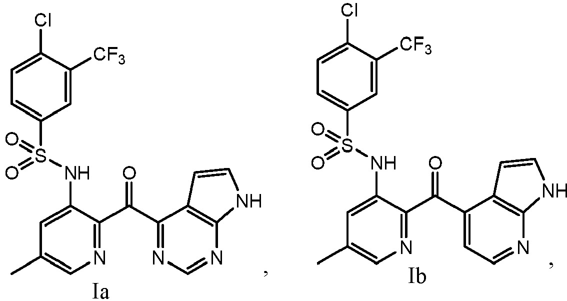 und 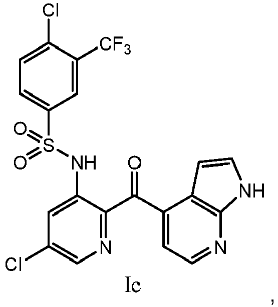 oder ein pharmazeutisch akzeptables Salz davon.

4. Die Verbindung zur Verwendung gemäß Anspruch 1, wobei die Verbindung 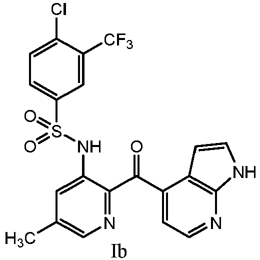 oder ein pharmazeutisch akzeptables Salz davon ist.

5. Die Verbindung zur Verwendung gemäß irgendeinem der Ansprüche 1 bis 4, zur Bekämpfung eines Bauchspeicheldrüsen-Adenokarzinoms.

6. Die Verbindung zur Verwendung gemäß irgendeinem der Ansprüche 1 bis 5, wobei die Verbindung der Formel I als eine pharmazeutische Zusammensetzung für orale Verabreichung bereitgestellt wird.

7. Die Verbindung zur Verwendung gemäß irgendeinem der Ansprüche 1 bis 6, wobei die wirksame Menge von 50 mg bis 300 mg beträgt, bevorzugt 150 mg.

8. Die Verbindung zur Verwendung gemäß irgendeinem der Ansprüche 1 bis 7, wobei die Verbindung einmal oder zweimal täglich verabreicht wird.

9. Die Verbindung zur Verwendung gemäß irgendeinem der Ansprüche 1 bis 8, wobei die Verwendung des Weiteren beinhaltet, dass ein oder mehrere zusätzliche therapeutische Wirkstoffe an den Patienten verabreicht werden, wobei der eine oder mehrere zusätzliche therapeutische Wirkstoff ausgewählt ist aus einem oder mehreren von einem Btk-Tyrosinkinaseinhibitor, Erbb2-Tyrosinkinaserezeptor-Inhibitor; Erbb4-Tyrosinkinaserezeptor-Inhibitor, mTOR-Inhibitor, Thymidylatsynthaseinhibitor, EGFR-Tyrosinkinaserezeptor-Inhibitor, Epidermal Growth Factor-Antagonist, Fyn-Tyrosinkinaseinhibitor, kit-Tyrosinkinaseinhibitor, Lyn-Tyrosinkinaseinhibitor, NK-Zellrezeptormodulator, PDGF-Rezeptorantagonist, PARP-Inhibitor, polyADP-Ribosepolymerase-Inhibitor, polyADP-Ribosepolymerase-1-Inhibitor, polyADP-Ribosepolymerase-2-Inhibitor, polyADP-Ribosepolymerase-3-Inhibitor, Galactosyltransferasemodulator, Dihydropyrimidindehydrogenase-Inhibitor, Orotatphosphoribosyltransferase-Inhibitor, Telomerasemodulator, Mucin-1-Inhibitor, Mucininhibitor, Secretinagonist, TNF-verwandter-Apoptose-induzierender-Ligand - Modulator, IL17-Gen-Stimulator, Interleukin 17E-Ligand, Neurokininrezeptoragonist, Cyclin G1-Inhibitor, Checkpoint-Inhibitor, PD-1-Inhibitor, PD-L1-Inhibitor, CTLA4-Inhibitor, Topoisomerase I-Inhibitor, Alk-5-Proteinkinase-Inhibitor,
Bindegewebswachstumsfaktor-Liganden-Inhibitor, Notch-2-Rezeptorantagonist, Notch-3-Rezeptorantagonist, Hyaluronidase-Stimulator, MEK-1-Proteinkinase-Inhibitor; MEK-2-Proteinkinase-Inhibitor, GM-CSF-Rezeptor-Modulator; TNF alpha-Ligand-Modulator, Mesothelin-Modulator, Asparaginase-Stimulator, Caspase-3-Stimulator; Caspase-9-Stimulator, PKN3-Gen-Inhibitor, Hedgehogprotein-Inhibitor; Smoothened-Rezeptorantagonist, AKT1-Gen-Inhibitor, DHFR-Inhibitor, Thymidinkinase-Stimulator, CD29-Modulator, Fibronectinmodulator, Interleukin-2-Ligand, Serinprotease-Inhibitor, D40LG-Gen-Stimulator; TNFSF9-Gen-Stimulator, 2-Oxoglutaratdehydrogenase-Inhibitor, TGF-beta-Typ II-Rezeptorantagonist, Erbb3-Tyrosinkinaserezeptor-Inhibitor, Cholecystokinin-CCK2-Rezeptorantagonist, Wilmstumor-Protein-Modulator, Ras GTPase-Modulator, Histondeacetylase-Inhibitor, Cyclin-abhängige-Kinase-4-Iinhibitor A-Modulator, Östrogenrezeptor-beta-Modulator, 4-1BB-Inhibitor, 4-1BBL-Inhibitor, PD-L2-Inhibitor, B7-H3-Inhibitor, B7-H4-Inhibitor, BTLA-Inhibitor, HVEM-Inhibitor, TIM3-Inhibitor, GAL9-Inhibitor, LAG3-Inhibitor, VISTA-Inhibitor, KIR-Inhibitor, 2B4-Inhibitor, CD160-Inhibitor und CD66e-Modulator.

10. Die Verbindung zur Verwendung gemäß Anspruch 9, wobei der eine oder mehrere zusätzliche therapeutische Wirkstoff FOLFIRINOX ist, oder wobei die eine oder mehrere zusätzlichen therapeutischen Wirkstoffe Gemcitabin und Paclitaxel sind.

11. Eine pharmazeutische Zusammensetzung zur Verwendung in der Behandlung von Bauchspeicheldrüsenkrebs oder bei der Bekämpfung eines Adenokarzinoms umfassend eine Verbindung wie in irgendeinem der Ansprüche 1 bis 5 definiert und einen pharmazeutisch akzeptablen Trägerstoff.

12. Die pharmazeutische Zusammensetzung zur Verwendung gemäß Anspruch 11 umfassend einen oder mehrere zusätzliche therapeutische Wirkstoffe zusätzlich zu der Verbindung wie in irgendeinem der Ansprüche 1 bis 5 definiert, wobei der eine oder mehrere zusätzliche therapeutische Wirkstoff FOLFIRINOX ist, oder wobei die eine oder mehrere zusätzlichen therapeutischen Wirkstoffe Gemcitabin und Paclitaxel sind.

## Revendications

1. Composé de Formule I : 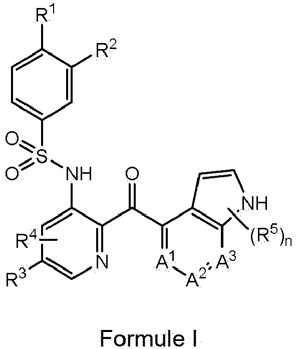 ou un sel pharmaceutiquement acceptable de celui-ci,
où
R¹ est un halogène ou un alkyle en C₁₋₆ ;
R² est un hydrogène, un halogène, un alkyle en C₁₋₆, un alcoxy en C₁₋₆, un haloalkyle en C₁₋₆, un haloalcoxy en C₁₋₆, ou -CN ;
R³ est un hydrogène, un halogène, ou un alkyle en C₁₋₆ ;
R⁴ est un hydrogène, un halogène, ou un alkyle en C₁₋₆ ;
chaque R⁵ est indépendamment un alkyle en C₁₋₆, -OH, ou -NH₂ ;
n vaut 0, 1, 2, ou 3 ; et
chacun parmi A¹, A², et A³ est -CH- ou -N-, où au moins un parmi A¹, A², ou A³ est -N-pour une utilisation dans le traitement du cancer du pancréas ou dans le contrôle d'un adénocarcinome.

2. Composé pour une utilisation selon la revendication 1, où
R¹ est un halogène ou un méthyle ;
R² est un halogène ou un haloalkyle en C₁₋₆ ;
R³ est un halogène ou un alkyle en C₁₋₆ ;
R⁴ est un hydrogène ;
n vaut 0 ;
A¹ est -CH- ou -N- ;
A² est -CH- ; et
A³ est -N-.

3. Composé pour une utilisation selon la revendication 1, où le composé est choisi dans le groupe constitué par : 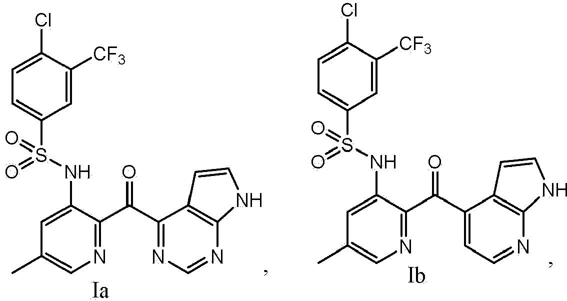 et 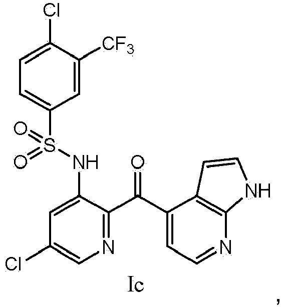 ou un sel pharmaceutiquement acceptable de celui-ci.

4. Composé pour une utilisation selon la revendication 1, où le composé est 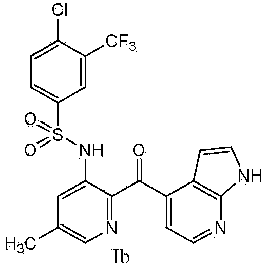 ou un sel pharmaceutiquement acceptable de celui-ci.

5. Composé pour une utilisation selon l'une quelconque des revendications 1 à 4, pour contrôler un adénocarcinome pancréatique.

6. Composé pour une utilisation selon l'une quelconque des revendications 1 à 5, où le composé de Formule 1 est fourni en tant que composition pharmaceutique pour une administration orale.

7. Composé pour une utilisation selon l'une quelconque des revendications 1 à 6, où la quantité efficace va de 50 mg à 300 mg, de préférence est de 150 mg.

8. Composé pour une utilisation selon l'une quelconque des revendications 1 à 7, où le composé est administré une fois par jour ou deux fois par jour.

9. Composé pour une utilisation selon l'une quelconque des revendications 1 à 8, l'utilisation comprenant en outre l'administration au patient d'un ou plusieurs composés thérapeutiques supplémentaires, où le ou les composés thérapeutiques supplémentaires sont choisis parmi un ou plusieurs éléments parmi un inhibiteur de tyrosine kinase Btk, un inhibiteur de récepteur tyrosine kinase Erbb2 ; un inhibiteur de récepteur tyrosine kinase Erbb4, un inhibiteur de mTOR, un inhibiteur de thymidylate synthase, un inhibiteur de récepteur tyrosine kinase EGFR, un antagoniste de facteur de croissance épidermique, un inhibiteur de tyrosine kinase Fyn, un inhibiteur de tyrosine kinase Kit, un inhibiteur de tyrosine kinase Lyn, un modulateur de récepteur de cellules NK, un antagoniste de récepteur PDGF, un inhibiteur de PARP, un inhibiteur de poly ADP ribose polymérase, un inhibiteur de poly ADP ribose polymérase 1, un inhibiteur de poly ADP ribose polymérase 2, un inhibiteur de poly ADP ribose polymérase 3, un modulateur de galactosyltransférase, un inhibiteur de dihydropyrimidine déshydrogénase, un inhibiteur d'orotate phosphoribosyltransférase, un modulateur de télomérase, un inhibiteur de mucine 1, un inhibiteur de mucine, un agoniste de sécrétine, un modulateur de ligand induisant l'apoptose lié au TNF, un stimulateur de gène IL17, un ligand d'interleukine 17E, un agoniste de récepteur de neurokinine, un inhibiteur de cycline G1, un inhibiteur de point de contrôle, un inhibiteur de PD-1, un inhibiteur de PD-L1, un inhibiteur de CTLA4, un inhibiteur de topoisomérase I, un inhibiteur de protéine kinase Alk-5, un inhibiteur de ligand du facteur de croissance du tissu conjonctif, un antagoniste de récepteur Notch-2, un antagoniste de récepteur Notch-3, un stimulateur de hyaluronidase, un inhibiteur de protéine kinase MEK-1 ; un inhibiteur de protéine kinase MEK-2, un modulateur de récepteur de GM-CSF, un modulateur de ligand de TNF alpha, un modulateur de mésothéline, un stimulateur d'asparaginase, un stimulateur de caspase-3, un stimulateur de caspase-9, un inhibiteur de gène PKN3, un inhibiteur de protéine hedgehog ; un antagoniste de récepteur Smoothened, un inhibiteur de gène AKT1, un inhibiteur de DHFR, un stimulateur de thymidine kinase, un modulateur de CD29, un modulateur de fibronectine, un ligand d'interleukine-2, un inhibiteur de protéase à sérine, un stimulateur de gène D40LG ; un stimulateur de gène TNFSF9, un inhibiteur de 2-oxoglutarate déshydrogénase, un antagoniste de récepteur TGF-bêta de type II, un inhibiteur de récepteur tyrosine kinase Erbb3, un antagoniste de récepteur CCK2 de cholécystokinine, un modulateur de protéine de tumeur de Wilms, un modulateur de GTPase Ras, un inhibiteur d'histone désacétylase, un modulateur d'inhibiteur A de kinase cycline-dépendant 4, un modulateur de récepteur bêta des œstrogènes, un inhibiteur de 4-1 BB, un inhibiteur de 4-1BBL, un inhibiteur de PD-L2, un inhibiteur de B7-H3, un inhibiteur de B7-H4, un inhibiteur de BTLA, un inhibiteur de HVEM, un inhibiteur de TIM3, un inhibiteur de GAL9, un inhibiteur de LAG3, un inhibiteur de VISTA, un inhibiteur de KIR, un inhibiteur de 2B4, un inhibiteur de CD160, et un modulateur de CD66e.

10. Composé pour une utilisation selon la revendication 9, où le ou les composés thérapeutiques supplémentaires sont le FOLFIRINOX, ou où le ou les composés thérapeutiques supplémentaires sont la gemcitabine et le paclitaxel.

11. Composition pharmaceutique pour une utilisation dans le traitement du cancer du pancréas ou dans le contrôle d'un adénocarcinome, comprenant un composé tel que défini dans l'une quelconque des revendications 1 à 5 et un excipient pharmaceutiquement acceptable.

12. Composition pharmaceutique pour une utilisation selon la revendication 11, comprenant un ou plusieurs composés thérapeutiques supplémentaires en plus du composé tel que défini dans l'une quelconque des revendications 1 à 5, où le ou les composés thérapeutiques supplémentaires sont le FOLFIRINOX, ou où le ou les composés thérapeutiques supplémentaires sont la gemcitabine et le paclitaxel.
